# EUROPEAN PATENT APPLICATION

(11) **EP 4 197 520 A1**
(43) Date of publication of application: **21.06.2023**
(21) Application number: 21214805.0
(22) Date of filing: 15.12.2021
(51) Int. Cl.: A61K 8/34, A61K 8/41, A61Q 5/12

(54) **AN AQUEOUS COMPOSITION FOR HAIR**

(71) Applicant: Kao Germany GmbH, 64297 Darmstadt (DE)
(72) Inventor: BAUER, Peter, 64297 Darmstadt (DE); BREAKSPEAR, Steven, 64297 DARMSTADT (DE); NÖCKER, Bernd, 64297 DARMSTADT (DE); UELLNER, Yuki, 64297 Darmstadt (DE)
(74) Representative: Grit, Mustafa

(57) **Abstract**

The present invention is on an aqueous composition for hair, especially human hair, for achieving long lasting conditioning. Accordingly, the present invention is an aqueous composition comprising one or more compounds selected from ortho diphenols substituted at p position with an alkyl or alkene having a length of 2 to 4 C atoms which is substituted with one or more of OH or NH2 or COOH group and has a pH equal or higher than 7.5 wherein the composition is non dyeing, non-oxidizing and non-reducing composition.

## Description

The present invention is on an aqueous composition for hair, especially human hair, for achieving long lasting conditioning.

Hair compositions have been used for many decades, primarily for improving hair properties. They are usually based on cationic compounds, which are adsorbed onto the hair surface via electrostatic interactions. Although the achieved results are generally satisfactory, the obtained effect lasts only for a very short time, as the adsorbed cationic compounds are washed off, mostly with a single shampooing, due to complexing with the anionic surfactants in the shampoo compositions. This requires the consumer to use conditioners after almost every wash cycle, which is often found time consuming, uneconomical, and not environmentally friendly.

There have been attempts to solve the problem by providing conditioners, which provide long lasting effects. One of the methods has been to use oily substances at high concentrations. Another method has been treating hair with cationic polymers and with heat as described in EP3313364, which produces long lasting conditioning effects, which may last for 5 to 10 washes.

Another proposed method in EP2056788 Unilever is using dihydroxy benzoic acid esterified poly(hydroxyethyl acrylate) or poly(hydroxyethyl methacrylate) polymers for the long lasting deposition of the polymers. The document only discloses dihydroxy phenolic compounds, which carries the reactive carboxyl group without a spacer alkyl chain.

The inventors of the present invention have found out that treating hair with an aqueous alkaline composition comprising one or more ortho diphenol substituted at p position prior to the application of a conditioner comprising one or more cationic compound provides hair long lasting conditioning. The conditioning effect measured in terms of reduced combing force and improves suppleness lasting 5 to 10 hair washes and hair keeps it improved properties at an acceptable level without using any additional hair conditioner.

Thus, the first object of the present invention is an aqueous composition comprising one or more compounds selected from ortho diphenols substituted at the p position with an alkyl or alkene having a length of 2 to 4 C atoms which is substituted with one or more of OH or NH2 or COOH group and has a pH equal to or higher than 7.5 wherein the composition is a non-dyeing, non-oxidizing and non-reducing composition.

The second object of the present invention is method of treating hair wherein the aqueous composition of the present invention is applied onto hair and the hair temperature is increased, preferably in the range of 60 to 140°C for a period of 1 to 60 min and afterwards hair is optionally rinsed off. It is noted that even after rinsing off the composition the long-lasting conditioning effect is achieved with a subsequent conditioner composition application.

The third object of the present invention is the use of the aqueous composition of the present invention for treating hair and especially for achieving long lasting conditioning on hair.

The fourth object of the present invention is a kit for hair comprising the aqueous composition of the present invention and a device for increasing hair temperature.

The term "long lasting" means that the conditioning effect achieved with the composition is not washed off with a single washing/shampooing but remains on the hair for at least 3 to 5 washes, preferably 5 to 7 hair washes.

The aqueous composition of the present invention comprises one or more compounds selected from ortho diphenols substituted at the p position with an alkyl or alkene having a C length of 2 to 4 which is substituted with one or more of OH or NH2 or COOH.

Suitable non-limiting examples are hydroxytyrosol, caffeic acid, dopamine, dihydroxyphenylalanine, noradrenalin, adrenalin, dihydroxy mandelic acid and norepinephrine. Preferred are dopamine, hydroxytyrosol, caffeic acid, dihydroxyphenylalanine and dihydroxy mandelic acid. More preferred are dopamine, hydroxytyrosol, caffeic acid and dihydroxy mandelic acid. The most preferred are dopamine, hydroxytyrosol and/or caffeic acid.

The composition comprises the compounds at a total concentration in the range of 0.01 to 10%, preferably 0.1 to 7.5%, more preferably 0.2 to 6% and most preferably 0.25 to 5% by weight, calculated to the total of the composition.

The aqueous composition of the present invention has an alkaline pH. The pH of the composition is in the range of 7.5 to 11.0, preferably 8.0 to 10.5, more preferably 8.5 to 10.5 and most preferably 8.5 to 10.0.

The alkalinity of the compositions is adjusted by adding an alkaline compound to the compositions and, therefore, the compositions comprise preferably an alkalizing agent. Suitable alkalizing agents are all known alkali metal hydroxides, and especially ammonia and alkyl and/or alkanol amines according to the general structure 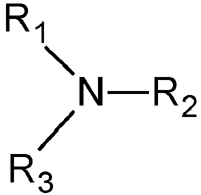 wherein R₁, R₂, and R₃ are same or different H, C₁ to C₄ alkyl which may be saturated or unsaturated, C₃ to C₄ branched alkyl which may as well be saturated or unsaturated, C₁ to C₄ hydroxyl alkyl, C₃ to C₄ saturated or unsaturated hydroxyl alkyl, C₃ to C₄ branched hydroxyl alkyl, with the condition that at least one of R₁, R₂, or R₃ is different from H. Suitable examples to alkyl or alkanol amines are monoethanolamine, diethanolamine, triethanolamine, monoethanol methylamine, monoethanoldimethylamine, di-ethanolmethylamine, monoethanolethylamine, monoethanoldiethylamine, diethanolethylamine, monoethanolpropylamine, monoethanoldipropylamine, diethanolpropylamine, monoethanolbutylamine, diethanolbutylamine and amino methyl propanol and their mixtures.

Preferred are ammonia and its salts, monoethanolamine, diethanolamine, triethanolamine, amino methyl propanol, and their mixtures.

The concentration of alkalizing agent is dependent on the selected pH and usually is in the range of 0.1 to 10%, preferably 0.1 to 7.5%, more preferably 0.25 to 6% and most preferably 0.5 to 5% by weight, calculated to the total of the composition.

The aqueous composition of the present invention is non dyeing, non-oxidizing and non-reducing composition. The term non-dyeing means that the composition does not have any dyeing effect on hair. Especially, it may as well be understood that the composition does not comprise any dyestuff either direct or oxidative dyes. This should be noted as some of the dyestuffs have been used especially for altering the colour of the compositions and not for adding color to hair and may suitably be understood within the scope of the invention.

The term non-oxidizing means that eh composition does not have any oxidizing effect and especially, does not comprise any oxidizing agent such as peroxides and per salts.

The term non-reducing means that the composition does not have any reducing effect on hair, especially, does not comprise any reducing agent.

The aqueous compositions may comprise additional compounds found customarily in cosmetic compositions. They may be preservatives, fragrances, solvents other than water such as alcohols ad other organic solvents which may be water miscible or immiscible, surfactants, polymers especially thickening polymers, humectants, and other available compounds for hair cosmetic compositions.

The aqueous composition is applied onto hair and preferably the hair temperature is increased for a period 1 to 60 min. The hair temperature may be adjusted according to the physical status of the hair in terms of damage level. The higher the hair damage, the lower the temperature, and to the contrary, the lower the hair damage, the higher the temperature. Usually, however, the temperature range should be selected between 60 and 230°C, preferably 80 to 230°C, more preferably 100 to 220°C and most preferably 120 to 180°C. The hair temperature may be measured with commercially available IR temperature measuring devices at a distance of approximately 5 to 10cm.

The hair is kept at the selected temperature for a period 1 to 60 min, preferably 1 to 45 min more preferably 2 to 30 min and most preferably 3 to 15 min. After the time is lapsed, the hair may be dried or first rinsed off and afterwards dried.

After processing of the aqueous composition of the present invention on hair, an aqueous hair conditioning composition comprising one or more hair conditioning compounds is applied onto hair. The application of the aqueous conditioning composition may be immediately after or after certain period has lapsed up to 48 hrs.

Hair temperature may be increased with a hair drier, with a flat or round iron or an iron of any other given shape, or any other equipment designed for this purpose.

The composition of the present invention is used for treating hair and especially for extending the conditioning effect of the conditioner applied onto hair subsequently. The conditioner comprises one or more cationic compounds. The conditioning composition may be an aqueous solution, aqueous dispersion, or an emulsion. The hair treated with the composition of the present invention is conditioned for a long period of time, at least for 3 to 5 hair washes, especially for 5 to 10 hair washes with a subsequently applied conditioner composition.

The composition of the present invention is provided as a kit for the users which comprises the aqueous composition of the present invention and a device for increasing hair temperature. Preferably, it comprises additional an aqueous hair conditioning composition.

The following examples is to illustrate the invention but not to limit.

### Example 1

To a bleached hair streak approximately 20 cm in length 3 g of a composition comprising 0.50%, by weight, dopamine was applied and left on the hair for approximately 60 min at ambient temperature. Afterwards the hair was dried and treated with an iron having a surface temperature of 170°C three times, each stroke taking approximately 5 seconds. Afterwards the hair streak was washed according to the standardized procedure as follows:
The hair steak was placed in a closable 45 ml test tube filled with a solution comprising 3.5% by weight of sodium laureth sulfate and having a pH 3.50. The tube was then placed in a shaking water bath at 40°C for 20 min which was shaken at a rate of 100 cycles per minute. The streak was then taken out and washed for 30 sec. with tap water at approximate 40°C. The streak was dried, and the combing force was measured. In case the streak was treated with multiple wash cycle, the above-described process was repeated.

The combing force was determined with a tensile testing machine from Zwick-Roell (Ulm, Germany) fitted with a combing attachment and using a nominal force of 20N. The combing rate was 15 seconds per 20 cm streak.

The results are presented in Table I.

| **Washing cyles** | **Combing force mN** |
|---|---|
| Prior to application | 137 |
| After application | 67 |
| 1x | 94 |
| 3x | 91 |
| 5x | 95 |
| 7x | 110 |

The above values are average of 20 measurements and the standard deviation was approximately +-10 mN. From the above results, it is beyond any doubt that the hair streak treated with dopamine solution has lower combing force compared to prior to treatment, which lasts for at least 7 hair washes.

### Example 2

A hair streak as described above was treated additionally with a commercially available hair after shampoo conditioner sold under the brand Goldwell Kerasilk Smooth Mask prior to treating the hair with a hair iron but after treating the hair with dopamine solution. The treatment with Kerasilk conditioner was carried out at ambient temperature and conditioner was left on the hair for 10 min and rinsed off afterwards with tap water at 40°C for 30 sec.

For comparative purposes the same hair streak was treated with only the Kerasilk conditioner.

The results are presented in Table II.

| | **Combing force mN** | |
|---|---|---|
| **Washing cyles** | **Kerasilk Mask** | **Kerasilk Mask+Dopamin** |
| Prior to application | 137 | 137 |
| After application | 51 | 45 |
| 1x | 75 | 60 |
| 3x | 88 | 65 |
| 5x | 103 | 72 |
| 7x | 105 | 82 |

The above values are average of 20 measurements and the standard deviation was approximately +-10 mN. From the above results it is beyond any doubt that the inventive composition provides additional benefit of reduced combing force for at least 7 hair washes, whereas the hair conditioned only with mask showed only slight long-lasting effect.

## Claims

1. An aqueous composition **characterized in that** it comprises one or more compounds selected from ortho diphenols substituted at p position with an alkyl or alkene having a C length of 2 to 4 which is substituted with one or more of OH or NH2 or COOH and has a pH equal or higher than 7.5, wherein the composition is non dyeing, non-oxidizing and non-reducing composition.

2. The composition according to claim 1 **characterized in that** it has a pH in the range of 7.5 to 11.0, preferably 8.0 to 10.5, more preferably 8.5 to 10.5.

3. The composition according to claim 1 and/or 2 **characterized in that** it has a pH in the range of 8.5 to 10.0.

4. The composition according to claims 1 to 3 **characterized in that** it comprises the ortho diphenol compounds at a total concentration in the range of 0.01 to 10%, preferably 0.1 to 7.5%, more preferably 0.2 to 6.0 % by weight, calculated to the total of the composition.

5. The composition according to claims 1 to 4 **characterized in that** it comprises the ortho diphenol compounds at a total concentration in the range of 0.25 to 5% by weight, calculated to the total of the composition.

6. The composition according to claims 1 to 5 **characterized in that** the ortho diphenol compound is selected from Hydroxytyrosol, caffeic acid, dopamine, dihydroxyphenylalanine, noradrenaline, adrenaline, 3,4-dihydroxy mandelic acid, dihydroxyphenylethylenglycol, and norepinephrine aldehyde.

7. The composition according to claims 1 to 6 **characterized in that** the ortho diphenol compound is Hydroxytyrosol or caffeic acid or dopamine.

8. The composition according to any of the preceding claims **characterized in that** the aqueous composition comprises one or more alkalizing agents.

9. The composition according to claim 8 **characterized in that** the alkalizing agent is selected from ammonia and/or its salts and alkanolamines and or their salts, and alkali hydroxides.

10. The composition according to claims 8 and/or 9 **characterized in that** the alkalizing agent is selected from ammonium hydroxide, ammonium chloride, monoethanolamine, diethanolamine, triethanolamine and amino methyl propanol.

11. The method of treating hair **characterized in that** the composition according to any of the preceding claims is applied onto hair and hair temperature is increased for a period 1 to 30 min and afterwards hair is optionally rinsed off.

12. The method according to claim 11 **characterized in that** the hair temperature is increased to 60 to 230°C, preferably 80 to 230°C, more preferably 100 to 220°C and most preferably 120 to 180°C.

13. The method according to claims 11 and/or 12 the hair temperature is increased to the required range with a hair dryer or with a flat or round iron or of any given shape.

14. Use of the aqueous composition according to the claims 1 to 10 for treating hair.

15. Kit for hair comprising the aqueous composition according to the claims 1 to 10 and a device for increasing the hair temperature.
